# FASCICULE DE BREVET EUROPEEN

(11) **EP 3 017 033 B1**
(45) Date de publication et mention de la délivrance du brevet: **12.02.2020**
(21) Numéro de dépôt: 14745206.4
(22) Date de dépôt: 03.07.2014
(51) Int. Cl.: C12M 1/00, C12N 1/06, B02C 17/16

(54) **PROCEDE OPTIMISE DE RUPTURE DES PAROIS DE CHLORELLES PAR BROYAGE MECANIQUE**
OPTIMIERTES VERFAHREN ZUM BRECHEN VON CHLORELLA-WÄNDEN DURCH MECHANISCHES ZERKLEINERN
OPTIMISED METHOD FOR BREAKING CHLORELLA WALLS BY MECHANICAL CRUSHING

(30) Priorité: 04.07.2013 FR 1356577
(43) Date de publication de la demande: 11.05.2016
(73) Titulaire: Corbion Biotech, Inc., South San Francisco, CA 94080 (US)
(72) Inventeur: TOURSEL, Béatrice, F-62920 Gonnehem (FR); DELANNOY, François, F-62157 Allouagne (FR); PATINIER, Samuel, F-59890 Quesnoy-sur-Deule (FR)
(74) Mandataire: Cabinet Becker et Associés
(86) Numéro de dépôt international: PCT/FR2014/051704
(87) Numéro de publication internationale: WO 2015/001261

(56) Documents cités:
- WO-A1-2012/109642
- GB-A- 1 432 039
- MOLINA GRIMA ET AL: "Downstream Processing of cell-mass and products" In: "HANDBOOK OF MICROALGAL CULTURE: BIOTECHNOLOGY AND APPLIED PHYCOLOGY", 1 janvier 2004 (2004-01-01), BLACKWELL/WILEY, XP008160049, ISBN: 978-0-632-05953-9 pages 215-251, page 232
- Anonymous: "Ceramic Grinding Beads", PROCESS TECHNOLOGIES FOR TOMORROW , 1 janvier 2006 (2006-01-01), XP055113545, Extrait de l'Internet: URL:http://imperia.mi-verlag.de/imperia/md /upload/product/8r25_hosokawa_power_beads_ engl.pdf [extrait le 2014-04-10]
- DOUCHA J ET AL: "Influence of processing parameters on disintegration of Chlorella cells in various types of homogenizers", APPLIED MICROBIOLOGY AND BIOTECHNOLOGY, vol. 81, no. 3, 29 août 2008 (2008-08-29), pages 431-440, XP019654147, SPRINGER, BERLIN, DE ISSN: 1432-0614, DOI: 10.1007/S00253-008-1660-6

## Description

La présente invention est relative à un procédé optimisé de rupture des parois cellulaires des microalgues du genre *Chlorella,* plus particulièrement *Chlorella vulgaris*, *Chlorella sorokiniana* ou *Chlorella protothecoides* à l'échelle industrielle.

### Présentation de l'état de l'art

Il est bien connu de l'homme du métier que les chlorelles sont une source potentielle de nourriture, car elles sont riches en protéines et autres nutriments essentiels.

Elles renferment notamment 45% de protéines, 20% de matières grasses, 20% de glucides, 5% de fibres et 10% de minéraux et de vitamines.

Pour utiliser efficacement *Chlorella* en Alimentation, il est souvent fait recours au « cassage cellulaire » de manière à faciliter sa digestibilité et son taux d'absorption.

Ce « cassage cellulaire » des microalgues est bien décrit dans la littérature brevets et non brevets par l'emploi de technologies variées :
- physiques (ultrasons, microbilles, chocs thermiques, haute pression...)
- chimiques (acides, alcalis, solvants organiques hydrophiles...)
- enzymatiques (cellulase, lipase...).

Ces technologies sont par exemple décrites dans les demandes de brevet ou brevets KR 2012/0064786, CN 101756300, JPH 0568536, JPS 4971187 ou US 5330913 ou dans des articles scientifiques comme ceux de SANDER & MURTHY, 2009, in ANASABE Annual International Meeting*,* ou ZHENG et al., 2011, in Applied Biochemistry, 164, 7, 1215-1224.

Cependant, il est généralement déploré que ces différentes voies mécaniques, chimiques ou enzymatiques sont peu extrapolables à l'échelle industrielle.

Les difficultés sont en effet importantes lorsqu'il faut traiter des biomasses de microalgues :
- de densité cellulaire élevée (> 100 g/l), et/ou
- dont la paroi cellulaire présente une résistance mécanique particulièrement élevée.

Ce qui est le cas pour la grande majorité des microalgues du genre *Chlorella.*

Le choix technologique des méthodes de cassage cellulaire devient plus limité encore lorsque, aux difficultés liées à la physiologie des microalgues, s'ajoutent des contraintes industrielles de type :
- haute capacité de production,
- fiabilité,
- coûts opérationnels,
- coûts d'investissement....

Finalement, à l'échelle industrielle, l'homme du métier privilégie les technologies de broyage mécanique au détriment des voies chimiques et enzymatiques, plus particulièrement avec deux options :
- le broyage par microbilles, ou
- la technologie de rupture à haute pression.

Quoi qu'il en soit, le broyage de microorganismes nécessite un apport énergétique conséquent et a donc un impact non négligeable sur le prix de revient.

Cela est particulièrement vrai sur des microalgues de type *Chlorella* qui présentent une paroi très résistante.

La technologie « Bead Mill » (broyeur horizontal à billes) constitue un choix technologique susceptible de répondre à ces différentes problématiques. Le document WO 2012109642 A décrit le broyage de microalgues dans un broyeur à billes.

Cependant son impact en matière de :
- dépenses d'investissement de capital, notamment en coûts de développement et de pièces non-consommables (acronyme « CAPEX ») et
- dépenses d'exploitation, notamment énergétiques (acronyme « OPEX »), est significatif et nécessite une optimisation pour améliorer les performances de l'installation.

L'ensemble de ces contraintes tant technologiques que financières constituent un frein aux développements de cette technologie « Bead Mill » à l'échelle industrielle.

### Objet de l'invention

Pour remédier à ces contraintes, la société Demanderesse a choisi de conduire ses travaux sur la maîtrise de la technologie de broyage à billes afin de parvenir à optimiser les coûts énergétiques (OPEX) ainsi que la capacité d'investissement nécessaire pour y parvenir (CAPEX).

Pour ce faire, la société Demanderesse a choisi de réaliser une étude d'évaluation, à l'échelle du laboratoire, de l'impact des paramètres clefs de conduite de cette technologie de broyage à billes, paramètres clefs qui conditionnent à l'échelle industrielle l'optimisation de l'OPEX et du CAPEX.

Ces paramètres clefs sont :
∘ la densité des billes,
∘ le diamètre des billes,
∘ le taux de remplissage de la chambre de broyage,
∘ le mode de passage,
∘ la vitesse périphérique des disques de broyage,
∘ la concentration cellulaire.

Ces travaux ont conduit la société Demanderesse à proposer un procédé de broyage mécanique, à l'échelle industrielle, de cellules de microalgues du genre *Chlorella,* broyage mécanique étant conduit dans un système de type Broyeur Horizontal à Billes, caractérisé en ce que :
- les billes présentent une densité apparente comprise entre 2 et 3,5 kg/l, et
- le taux de remplissage de la chambre de broyage est supérieur à 80 %.

De préférence, les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

De manière préférentielle, le procédé conforme à l'invention comprend :
- l'utilisation de billes en silicate de zirconium, et/ou
- un taux de remplissage supérieur ou égal à 85 %.

Un premier mode de réalisation du procédé conforme à l'invention comprend la conduite du broyage en mode continu, par passages successifs en série.

Un deuxième mode de réalisation du procédé conforme à l'invention comprend l'utilisation de billes présentant un diamètre inférieur à 1 mm, de préférence inférieur à 0,8 mm.

La société Demanderesse a par ailleurs trouvé qu'une combinaison particulière des paramètres de conduite du broyage mécanique des microalgues du genre *Chlorella,* en particulier *Chlorella protothecoides,* pouvait permettre d'optimiser l'OPEX au détriment du CAPEX, et réciproquement.

La société Demanderesse offre ainsi des variantes de son procédé conforme à l'invention permettant à l'homme du métier de choisir sa configuration en regard de son cahier des charges (gain en termes d'énergie de consommation ou de coût d'investissement dans le matériel).

C'est ainsi que pour optimiser l'OPEX, la société Demanderesse a trouvé qu'une première variante peut être de modérer la densité des microalgues à broyer, à un taux par exemple inférieur à 250 g/l.

Une seconde variante peut-être le choix d'une vitesse périphérique des disques de broyage modérée, par exemple inférieure à 10 m/s.

Sans que ces deux variantes ne soient mutuellement exclusives. Le procédé peut présenter ces deux variantes ou l'une des deux.

Pour optimiser le CAPEX, la société Demanderesse a trouvé qu'il fallait au contraire augmenter la densité cellulaire des microalgues à broyer, à un taux par exemple supérieur à 250 g/l.

Une seconde variante peut-être le choix d'une vitesse périphérique des disques de broyage accentuée, par exemple supérieure à 10 m/s.

Ici encore, sans que ces deux variantes ne soient mutuellement exclusives. Le procédé peut présenter ces deux variantes ou l'une des deux.

### Description détaillée de l'invention

La société Demanderesse a trouvé que l'exploitation raisonné de la technologie de broyage mécanique pour rompre la paroi de microalgues du genre *Chlorella,* à l'aide d'un broyeur horizontal à billes, permet de parvenir à un taux de broyage souhaité en optimisant les coûts énergétiques (OPEX) et coûts d'investissement (CAPEX).

L'amélioration de la performance globale de l'installation repose ainsi sur le choix de paramètres précis qui permettent d'optimiser l'OPEX et CAPEX ou de trouver le meilleur compromis.

Le procédé, conforme à l'invention, de broyage mécanique, à l'échelle industrielle, de cellules de microalgues du genre *Chlorella,* broyage mécanique conduit dans un système de type Broyeur Horizontal à Billes, est donc caractérisé en ce que :
- les billes présentent une densité apparente comprise entre 2 et 3,5 kg/l, et
- le taux de remplissage de la chambre de broyage est supérieur ou égal à 80 %.

### Densité apparente des billes

Comme il sera exemplifié ci-après, les billes de silicate de zirconium présentent les meilleures performances pour cette application en limitant l'énergie spécifique requise pour parvenir au taux de broyage ciblé. Ainsi, la densité apparente est comprise entre 2 et 3,5 kg/l, de préférence entre 2 et 3,2 kg/l.

### Taux de remplissage

Le taux de remplissage est défini comme étant le volume vide de la chambre de broyage moins le volume occupé par le système d'agitation (généralement défini par le fournisseur).

L'énergie spécifique à taux de remplissage plus faible est légèrement plus élevée qu'à haut taux de remplissage. De plus, la productivité y est améliorée.

En conséquence, de manière préférentielle, le procédé conforme à l'invention comprend :
- l'utilisation de billes en silicate de zirconium, et/ou
- un taux de remplissage supérieur ou égal à 85 %.

De préférence, le procédé utilise des billes en silicate de zirconium et un taux de remplissage supérieur à 85 %.

Le taux de remplissage peut être compris entre 80 et 95 %, notamment entre 85 et 90 %
**Un premier mode de réalisation du procédé conforme à l'invention** comprend la conduite du broyage en mode continu, par passages successifs en série.

L'opération de broyage à billes est classiquement réalisée selon plusieurs schémas de fonctionnement : recirculation, monopassage, multipassages...

Dans une configuration industrielle, un système continu est préféré et implique un mode de fonctionnement en monopassage ou multipassages.

Comme il sera exemplifié ci-après, la performance est améliorée en augmentant le nombre de passages puisque qu'un taux de broyage plus élevé est obtenu à temps de séjour équivalent et sans augmentation significative de l'énergie spécifique.

Dans le procédé conforme à l'invention, un mode en multipassages (plusieurs broyeurs en série) sera préféré pour l'optimisation du procédé à l'échelle industrielle. Notamment, le procédé peut comprendre 2, 3, 4, 5 passages ou plus.

**Un deuxième mode de réalisation du procédé conforme à l'invention** comprend l'utilisation de billes présentant un diamètre inférieur à 1 mm, de préférence inférieur à 0,8 mm. Dans un mode de réalisation particulier, les billes ont un diamètre compris entre 0,3 et 0,8 mm.

L'impact du diamètre des billes de broyage est étudié sur une même gamme en silicate de zirconium (billes de même densité) avec un diamètre variant de 0,3 mm à 1,7 mm.

Comme il sera exemplifié ci-après, les billes de faible diamètre permettent une meilleure performance énergétique ainsi qu'une meilleure productivité.

Pour permettre à l'homme du métier de choisir sa configuration en regard de son cahier des charges (favoriser l'OPEX ou le CAPEX), la société Demanderesse offre des variantes de son procédé conforme à l'invention.

La société Demanderesse a trouvé que ces options pouvaient être proposés en agissant sur :
- la densité cellulaire des microalgues du genre *Chlorella* à broyer,
- la vitesse périphérique des disques de broyage.

### Densité cellulaire

L'impact de la concentration cellulaire de la biomasse sur les performances de broyage est évalué en choisissant une biomasse issue d'un même lot de production, préparée à plusieurs concentrations (20 %, 25,2 % et 32,9 %).

Le broyage est effectué dans les mêmes conditions et les résultats sont comparés relativement à la matière sèche des échantillons ainsi générés.

L'interprétation des résultats est réalisée sous deux angles différents :
- en analysant l'impact de la concentration cellulaire sur l'énergie spécifique de broyage requise pour obtenir un taux de broyage défini,
- en analysant sous l'angle de la productivité, le débit d'introduction de la biomasse (relativement à la biomasse sèche) en regard du taux de broyage ciblé.

### Vitesse périphérique des disques de broyage

L'optimisation du broyage nécessite de définir également la vitesse périphérique optimale des disques de broyage.

L'interprétation des résultats est également réalisée sous deux angles différents :
- en analysant l'énergie spécifique de broyage en fonction de la vitesse périphérique,
- en analysant sous l'angle de la productivité, selon la vitesse périphérique, le débit nécessaire pour parvenir au taux de broyage ciblé.

Par ailleurs, des problématiques d'abrasion des disques et de la chambre de broyage ainsi que les problématiques d'usure des billes sont à considérer puisque pouvant avoir un impact économique non négligeable sur le procédé.

La société Demanderesse recommande alors de limiter la vitesse périphérique à une valeur inférieure à 15m/s (voire 13 m/s) pour éviter une abrasion excessive.

C'est ainsi que dans le cas du broyage d'une Chlorelle, en particulier de type *Chlorella protothecoides,* pour optimiser l'OPEX, la société Demanderesse a trouvé qu'une première variante peut être :
- de modérer la densité des microalgues à broyer à un taux inférieur à 250 g/l et/ou
- de choisir une vitesse périphérique des disques de broyage inférieure à 10 m/s.

De préférence, la densité des microalgues à broyer est comprise supérieure ou égale à 150 g/l et inférieure 250 g/l. De préférence, la vitesse périphérique des disques de broyage est supérieure ou égale à 6 m/s et inférieure à 10 m/s.

Pour optimiser le CAPEX, dans le cas du broyage d'une Chlorelle, en particulier de type *Chlorella protothecoides,* la société Demanderesse a trouvé qu'il fallait au contraire :
- augmenter la densité cellulaire des microalgues à broyer à un taux supérieur à 250 g/l et/ou
- choisir une vitesse périphérique des disques de broyage supérieure à 10 m/s.

De préférence, la densité des microalgues à broyer est comprise supérieure à 550 g/l et inférieure ou égale à 350 g/l. Notamment, la vitesse périphérique des disques de broyage peut être comprise entre 10 m/s et 15 m/s, de préférence entre 11 m/s et 13 m/s.

Bien entendu, la présente invention est relative à toute combinaison de modes de réalisation décrits dans la présente demande.

Par « échelle industrielle » est de préférence entendu un procédé dans lequel :
- le volume de la chambre de broyage est supérieur ou égal à 100 litres, de préférence à 500 litres ; et/ou
- le débit est supérieur à 1 m³/h ; et/ou
- un batch de 1 à 200 m³.

L'invention sera mieux comprise à l'aide des exemples qui suivent, lesquels se veulent illustratifs et non limitatifs.

### EXEMPLES

### Exemple 1. Préparation d'une biomasse de microalgues Chlorella protothecoides et présentation des outils mis en œuvre

Le protocole de fermentation est adapté de celui décrit en toute généralité dans la demande de brevet WO 2010/120923.

Le fermenteur de production est inoculé avec une préculture de *Chlorella protothecoides.* Le volume après inoculation atteint 9 000 L.

La source de carbone employée est un sirop de glucose 55 % p/p stérilisé par application d'un barème temps/température.

La fermentation est un « fed-batch » durant lequel le débit de glucose est ajusté de sorte à maintenir une concentration de glucose résiduel de 3 à 10 g/L.

La durée du fermenteur de production est de 4 à 5 jours.

En fin de fermentation, la concentration cellulaire atteint 185 g/L.

Pendant la phase d'alimentation en glucose, la teneur en azote dans le milieu de culture est limitée pour permettre l'accumulation de lipides à hauteur de 50 % (en poids de biomasse).

La température de fermentation est maintenue à 28°C

Le pH de fermentation avant inoculation est ajusté à 6,8 puis est régulé sur cette même valeur pendant la fermentation.

L'oxygène dissous est maintenu à un minimum de 30 % en contrôlant l'aération, la contre pression et l'agitation du fermenteur.

Le moût de fermentation est traité thermiquement sur une zone HTST avec un barème de 1 min à 75°C et refroidi à 6°C.

La biomasse est alors lavée à l'eau potable décarbonatée avec un ratio de dilution de 6 pour 1 (eau/moût) et concentrée à 250 g/L (25% DCW « *Dry Cell Weight* ») par centrifugation en utilisant une Alfa Laval FEUX 510.

### Technologie de broyage à billes

La biomasse de fermentation ainsi préparée est utilisée pour réaliser un criblage des paramètres de broyage sur broyeur à billes de laboratoire type « Bead Mill » :

| Type de Machine | LabStar LS1 |
|---|---|
| Equipement | LME |
| Volume de chambre (litre) | 0,7 |
| Vitesse périphérique (m/s) | 8 à 12 |
| Débit alimentation (kg/h) | 10 à 90 |
| Matériaux des billes | Verre |
| | Silicate de Zirconium |
| | Oxyde de Zirconium |
| Diamètre de billes (mm) | 0,3 à 1,7 |
| Taux de remplissage (%) | 80 à 90% |

Les résultats des paramètres étudiés sont caractérisés par plusieurs points obtenus par plusieurs passages successifs à débit constant.

Les courbes ainsi obtenues pour chaque série de paramètres testés sont alors comparées de façon relative entre elles en termes d'énergie spécifique ou de productivité.

Par ailleurs, pour l'étude d'un paramètre spécifique, la biomasse utilisée pour l'essai est issue du même lot afin de s'affranchir de la variabilité de la composition d'un lot à l'autre.

### Mesure du taux de cassage cellulaire :

La mesure du taux de broyage est effectuée par comptage microscopique des cellules résiduelles après broyage relativement à l'échantillon initial de référence.

Les échantillons sont dilués au 1/800. L'analyse est effectuée par comptage sur cellule de Malassez selon la méthode d'utilisation standard au microscope optique au grossissement 10x40.

Le taux de cassage cellulaire est déterminé par calcul du pourcentage de cellules résiduelles par rapport à l'échantillon initial de référence.

### Exemple 2. Optimisation des paramètres de broyage

### Densité des billes

L'impact de la densité des billes de broyage est étudié à partir d'une sélection de billes de matériaux ayant des densités différentes mais de même diamètre (0,6 mm) :

| **Billes** | Verre | | ZS (Zirconium Silicate) | | ZO (Zirconium Oxide) | |
|---|---|---|---|---|---|---|
| **Densité (kg/L)** | 2.5 | | 4 | | 6 | |
| **Densité apparente (kg/L)** | 1.5 | | 2.5 | | 3.6 | |
| **Composition chimique** | SiO₂ (%) | 72,5 | ZrO₂ (%) | 55-65 | ZrO₂ + HfO₂ (%) | 95 |
| | Na₂O (%) | 13 | SiO₂ (%) | 35-40 | Y₂O₃ (%) | 5 |
| | CaO (%) | 9 | | | | |
| | MgO (%) | 4 | | | | |

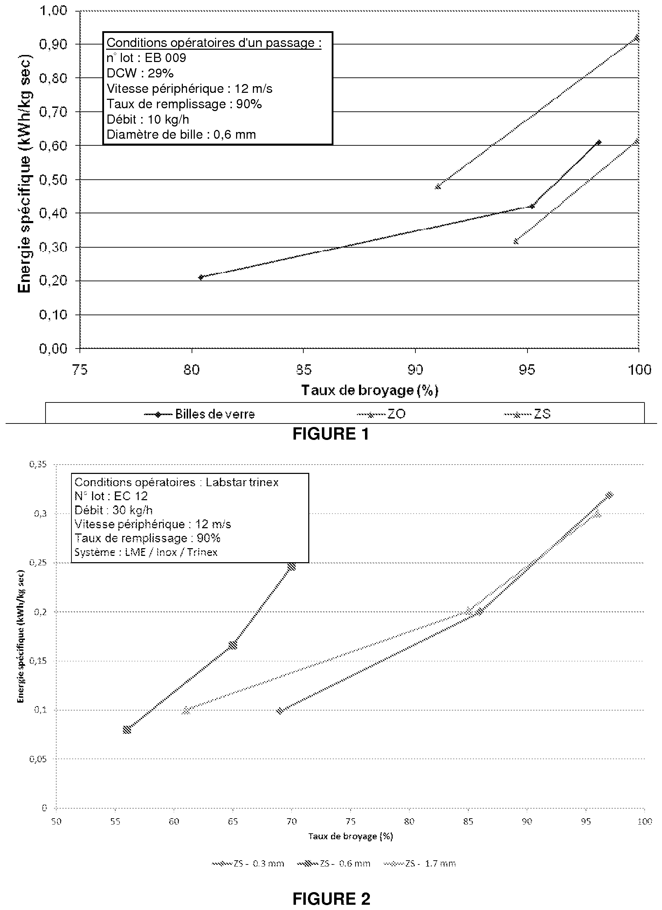

La comparaison des résultats obtenus, présentés dans la **Figure 1**, à taux de broyage équivalent selon le matériau des billes utilisées dans les mêmes conditions opératoires met en évidence une différence significative de l'énergie spécifique consommée.

Les billes de forte densité (ZO - oxyde de zirconium) entrainent une consommation en énergie spécifique nettement plus importante que les billes de silicate de zirconium.

Bien que les billes de verre aient une densité plus faible, les performances obtenues sont moins bonnes, ce qui nécessite un nombre de passages plus important pour parvenir au taux de broyage ciblé générant une consommation en énergie spécifique supérieure.

Les billes de silicate de zirconium présentent les meilleures performances pour cette application en limitant l'énergie spécifique requise pour parvenir au taux de broyage ciblé.

### Diamètre des billes

L'impact du diamètre des billes de broyage est étudié sur une même gamme en silicate de zirconium (billes de même densité) avec un diamètre variant de 0,3 mm à 1,7 mm.

Les résultats obtenus, présentés dans la **Figure 2**, dans les mêmes conditions opératoires en variant uniquement le diamètre des billes, mettent en évidence un impact fort sur les performances de broyage de ce paramètre.

A diamètre élevé (ici 1,7 mm), le nombre de passages ainsi que l'énergie spécifique requise pour parvenir au taux de broyage ciblé est significativement plus élevé qu'à diamètre faible.

La différence entre les billes de 0,3 mm et 0,6 mm est moins significative. Les billes de faible diamètre permettent une meilleure performance énergétique ainsi qu'une meilleure productivité.

### Taux de remplissage de la chambre

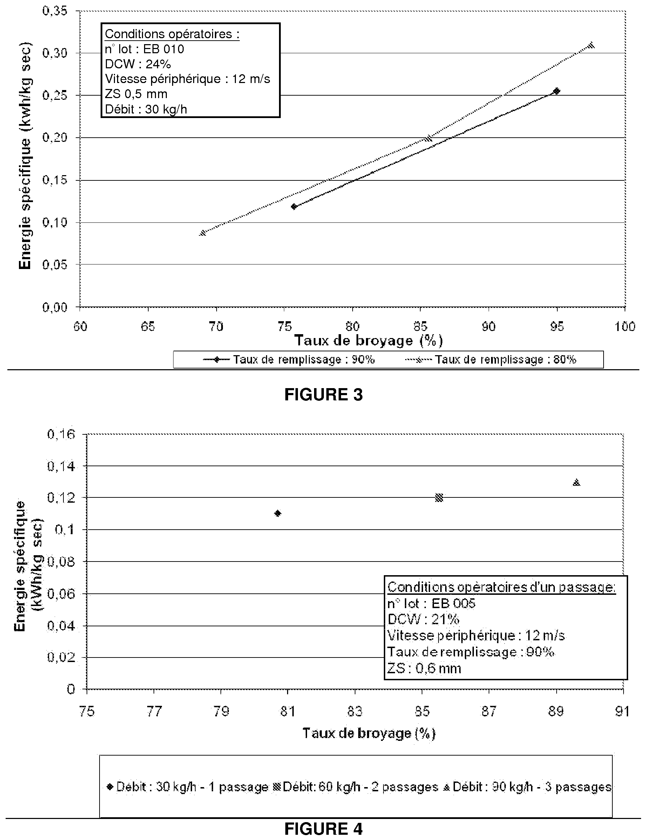

Comme le montre la **Figure 3**, l'énergie spécifique à taux de remplissage faible est légèrement plus élevée que lorsque le taux de remplissage est élevé.

De plus, la productivité y est améliorée (dans les mêmes conditions opératoires, le taux de broyage obtenu au 1^{er} et 2^{ème} passage est plus élevé à 90 % qu'à 80 % de taux de remplissage).

### Schéma de fonctionnement - impact du mode de passage

Comme le montre la **Figure 4**, en termes de temps de séjour dans la chambre de broyage, les trois schémas de fonctionnement testés (monopassage, multipassages) sont équivalents (1 passage à 30kg/h / 2 passages à 60kg/h / 3 passages à 90kg/h).

Par ailleurs, l'énergie spécifique de broyage est presque équivalente pour ces trois schémas.

Cependant, la performance est améliorée en augmentant le nombre de passages puisque qu'un taux de broyage plus élevé est obtenu à temps de séjour équivalent et sans augmentation significative de l'énergie spécifique.

Un mode en multipassages (plusieurs broyeurs en série) sera donc préféré pour l'optimisation du procédé à l'échelle industrielle.

### Vitesse périphérique des disques de broyage

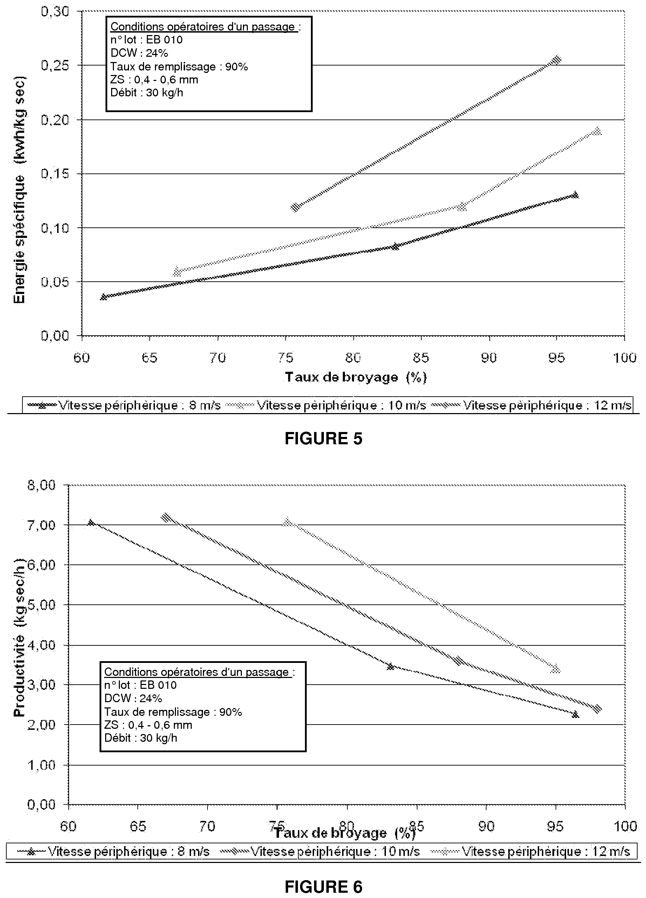

Selon la **Figure 5** : En analysant relativement l'énergie spécifique de broyage en fonction de la vitesse périphérique, la position relative des courbes entre-elles met en évidence qu'à taux de broyage défini, un fonctionnement à haute vitesse périphérique nécessite une énergie spécifique significativement plus élevée.

Le broyage à vitesse périphérique modéré permet donc d'atteindre de meilleures performances énergétiques

Selon la **Figure 6** : En analysant sous l'angle de la productivité, à vitesse périphérique plus élevée, un débit supérieur est obtenu pour parvenir au taux de broyage ciblé.

La productivité d'une installation sera plus élevée lorsque la vitesse périphérique est augmentée.

Pour optimiser une installation industrielle, un compromis est à définir entre les coûts opérationnels (OPEX) optimisés à vitesse périphérique modéré et les dépenses d'investissement (CAPEX) minimisée à haute vitesse périphérique.

Par ailleurs, des problématiques d'abrasion des disques et de la chambre de broyage ainsi que les problématiques d'usure des billes sont à considérer puisque pouvant avoir un impact économique non négligeable sur le procédé.

Il est préféré de limiter la vitesse périphérique à une valeur inférieure à 15 m/s (voire 13 m/s) pour éviter une abrasion excessive.

### Densité cellulaire

Outre les paramètres physiques de l'installation de broyage à billes, certains critères sur la biomasse à broyer peuvent avoir un impact non négligeable sur les performances de broyage.

Ainsi, l'impact de la concentration cellulaire de la biomasse sur les performances de broyage est évalué.

Une biomasse issue d'un même lot de production est préparée à plusieurs concentrations (20 %, 25,2 % et 32,9 %).

Le broyage est effectué dans les mêmes conditions et les résultats sont comparés relativement à la matière sèche des échantillons ainsi générés.

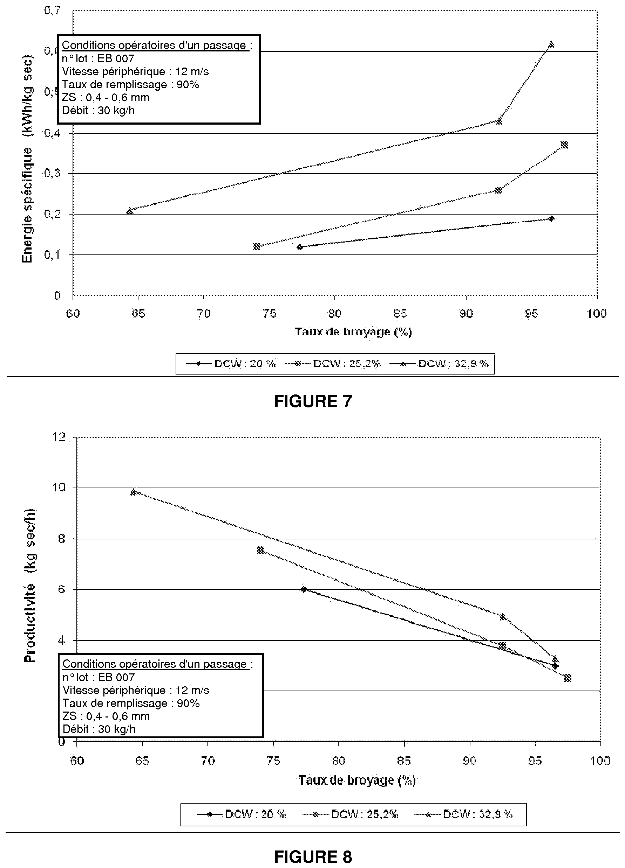

Selon la **Figure 7** : En analysant relativement l'impact de la concentration cellulaire sur l'énergie spécifique de broyage requise pour obtenir un taux de broyage défini, des différences significatives sont mises en évidence.

A haute concentration cellulaire, l'énergie spécifique consommée à un taux de broyage donné est nettement supérieure qu'à plus faible concentration cellulaire.

Le broyage d'une biomasse cellulaire à concentration modérée permet donc d'atteindre de meilleures performances énergétiques qu'à haute concentration.

Selon la **Figure 8** : En analysant sous l'angle de la productivité, à haute concentration cellulaire, une installation équivalente permettra de passer un débit supérieur tout en parvenant au taux de broyage ciblé.

Autrement dit, la productivité d'une installation sera plus élevée lorsque la concentration cellulaire est augmentée.

### Description des Figures

**FIGURE 1** : Impact de la densité des billes (diamètre 0,6 mm) sur les performances de broyage
**FIGURE 2** **:** Impact du diamètre des billes (ZS) sur les performances de broyage
**FIGURE 3** **:** Impact du taux de remplissage de la chambre sur les performances de broyage
**FIGURE 4** : Impact du mode de passage sur les performances de broyage
**FIGURE 5** **:** Impact de la vitesse périphérique sur l'énergie spécifique de broyage
**FIGURE 6** **:** Impact de la vitesse périphérique sur la productivité de l'opération de broyage
**FIGURE 7** **:** Impact de la concentration cellulaire sur l'énergie spécifique de broyage
**FIGURE 8** **:** Impact de la concentration cellulaire sur la productivité de l'opération de broyage

## Revendications

1. Procédé de broyage mécanique, à l'échelle industrielle, de cellules de microalgues du genre *Chlorella*, le broyage mécanique étant conduit dans un système de type Broyeur Horizontal à Billes, **caractérisé en ce que** :
- les billes en silicate de zirconium présentent une densité apparente comprise entre 2 et 3,5 kg/l,
- le taux de remplissage de la chambre de broyage est supérieur à 80 %, de préférence supérieur ou égal à 85 %, et
- le broyage mécanique est conduit en mode continu par passages successifs en série.

2. Procédé selon la revendication 1, **caractérisé en ce que** les microalgues du genre Chlorella sont choisies dans le groupe constitué de *Chlorella vulgaris*, *Chlorella sorokiniana* et *Chlorella protothecoides,* et sont plus particulièrement *Chlorella protothecoides.*

3. Procédé selon l'une quelconque des revendications 1 à 2, **caractérisé en ce que** les billes présentent un diamètre inférieur à 1 mm, de préférence inférieur à 0,8 mm.

4. Procédé selon l'une quelconque des revendications 1 à 3 pour optimiser les couts énergétiques d'une installation industrielle de broyage de microalgues du type *Chlorella*, en particulier *Chlorella protothecoides,* **caractérisé en ce qu'**il comprend:
- la modération de la densité des microalgues à broyer à un taux inférieur à 250 g/l et/ou
- le choix d'une vitesse périphérique des disques de broyage inférieure à 10 m/s.

5. Procédé selon l'une quelconque des revendications 1 à 4 pour optimiser la capacité d'investissement d'une installation industrielle de broyage de microalgues du type *Chlorella,* en particulier *Chlorella protothecoides,* **caractérisé en ce qu'**il comprend:
- l'augmentation de la densité cellulaire des microalgues à broyer à un taux supérieur à 250 g/l et/ou
- le choix d'une vitesse périphérique des disques de broyage supérieure à 10 m/s.

## Patentansprüche

1. Verfahren zur mechanischen Zerkleinerung im großtechnischen Maßstab von Zellen von Mikroalgen der Gattung *Chlorella,* wobei die mechanische Zerkleinerung in einem System des Typs Horizontalperlmühle durchgeführt wird, **dadurch gekennzeichnet, dass**
- die Zirconiumsilikat-Perlen eine Schüttdichte zwischen 2 und 3,5 kg/l aufweisen,
- der Füllungsgrad der Zerkleinerungskammer größer 80%, vorzugsweise größer oder gleich 85% ist, und
- die mechanische Zerkleinerung in kontinuierlicher Weise mittels serieller aufeinanderfolgender Durchgänge durchgeführt wird.

2. Verfahren gemäß Anspruch 1, **dadurch gekennzeichnet, dass** die Mikroalgen der Gattung *Chlorella* aus der Gruppe ausgewählt sind, bestehend aus *Chlorella vulgaris*, *Chlorella sorokiniana* und *Chlorella protothecoides*, und insbesondere *Chlorella protothecoides* sind.

3. Verfahren gemäß einem der Ansprüche 1 bis 2, **dadurch gekennzeichnet, dass** die Perlen einen Durchmesser kleiner als 1 mm, vorzugsweise kleiner als 0,8 mm aufweisen.

4. Verfahren gemäß einem der Ansprüche 1 bis 3 zur Optimierung der Energiekosten einer großtechnischen Anlage zur Zerkleinerung von Mikroalgen des Typs *Chlorella,* insbesondere *Chlorella protothecoides,* **dadurch gekennzeichnet, dass** es umfasst:
- die Verringerung der Dichte der Mikroalgen, die zerkleinert werden sollen, auf einen Wert unter 250 g/l, und/oder
- die Wahl einer Umfangsgeschwindigkeit der Zerkleinerungsscheiben unter 10 m/s.

5. Verfahren gemäß einem der Ansprüche 1 bis 4 zur Optimierung der Investitionskapazität einer großtechnischen Anlage zur Zerkleinerung von Mikroalgen des Typs *Chlorella,* insbesondere *Chlorella protothecoides,* **dadurch gekennzeichnet, dass** es umfasst:
- die Erhöhung der Zelldichte der Mikroalgen, die zerkleinert werden sollen, auf einen Wert über 250 g/l, und/oder
- die Wahl einer Umfangsgeschwindigkeit der Zerkleinerungsscheiben über 10 m/s.

## Claims

1. A process for the mechanical milling, on an industrial scale, of cells of microalgae of the Chlorella genus, the mechanical milling being carried out in a system of horizontal bead mill type, **characterized in that**:
- the zirconium silicate beads have an apparent density of between 2 and 3.5 kg/l,
- the filling rate of the milling chamber is greater than 80%, preferably greater than or equal to 85%, and
- the mechanical milling is carried out in continuous mode by means of a series of successive passes.

2. The process as claimed in claim 1, **characterized in that** the microalgae of the *Chlorella* genus are chosen from the group consisting of *Chlorella vulgaris*, *Chlorella sorokiniana* and *Chlorella protothecoides,* and are more particularly *Chlorella protothecoides.*

3. The process as claimed in either one of claims 1 and 2, **characterized in that** the beads have a diameter of less than 1 mm, preferably less than 0.8 mm.

4. The process as claimed in any one of claims 1 to 3, for optimizing the energy costs of an industrial facility for milling microalgae of the *Chlorella* type, in particular *Chlorella protothecoides,* **characterized in that** it comprises:
- moderating the density of the microalgae to be milled to a level of less than 250 g/l and/or
- choosing a peripheral speed of the milling disks of less than 10 m/s.

5. The process as claimed in any one of claims 1 to 4, for optimizing the investment capacity of an industrial facility for milling microalgae of the *Chlorella* type, in particular *Chlorella protothecoides,* **characterized in that** it comprises:
- increasing the cell density of the microalgae to be milled to a level of greater than 250 g/l and/or
- choosing a peripheral speed of the milling disks of greater than 10 m/s.
